Europäisches Patentamt

European Patent Office

Office européen des brevets

(11) Veröffentlichungsnummer: **0 447 745 A2**

# EUROPÄISCHE PATENTANMELDUNG

(21) Anmeldenummer: 91100310.1

(22) Anmeldetag: 11.01.91

(51) Int. Cl.5: **A61M 36/12, A61N 5/10**

(30) Priorität: 06.02.90 DE 4003458

(43) Veröffentlichungstag der Anmeldung:
25.09.91 Patentblatt 91/39

(84) Benannte Vertragsstaaten:
FR IT NL

(71) Anmelder: **ISOTOPEN-TECHNIK DR. SAUERWEIN GMBH**
**Bergische Strasse 16**
**W-5657 Haan/Rheinl. 1(DE)**

(72) Erfinder: **Fritz, Peter, Dr.Med.**
**Im Neuen Heimer Feld 400**
**W-6900 Heidelberg 1(DE)**

(74) Vertreter: **König, Reimar, Dr.-Ing. et al**
**Patentanwälte Dr.-Ing. Reimar König**
**Dipl.-Ing. Klaus Bergen Wilhelm-Tell-Strasse**
**14 Postfach 260162**
**W-4000 Düsseldorf 1(DE)**

(54) Vorrichtung zum Behandeln des Körperinneren mit radioaktiver Strahlung.

(57) Vorrichtung zur Behandlung des Körperinneren mit radioaktiver Strahlung mittels einer ferngesteuerten Belade- und/oder Lagerstation für eine oder mehrere Strahlenquellen (2), die über eine Fördervorrichtung und einen Ausfahrschlauch (1) zur Behandlungsstelle verfahren werden. Die Vorrichtung dient insbesondere zum endoluminalen Bestrahlen eines zentralen Bronchialkarzinoms und nimmt als Applikator die aus dem ferngesteuerten Beladegerät verfahrene Strahlenquelle (2) auf; sie umfaßt einen oder mehrere im Bereich der Strahlenquelle (2) angeordnete, einen axialen Durchlaß für die Atmung freihaltende, eine Verbesserung der Lage der Strahlenquelle (optimale Zentrierung) bewirkende Spreizköpfe (4, 5, 6). Die hieraus resultierende der Trachealwand oder Bronchialwand angepaßte gleichmäßigere Isodosenverteilung, schützt die Bronchialwand vor steilen Dosisgradienten. Auf diese Weise lassen sich Komplikationen durch eine zu hohe Strahlendosis vermeiden.

Fig.1

# VORRICHTUNG ZUM BEHANDELN DES KÖRPERINNEREN MIT RADIOAKTIVER STRAHLUNG

Die Erfindung betrifft eine Vorrichtung zum Behandeln des Körperinneren mit radioaktiver Strahlung mit in einem Ausfahrschlauch zum Behandlungsort verfahrbaren Strahlenquelle, insbesondere zum endoluminalen Bestrahlen eines zentralen Bronchialkarzinoms.

Eine derartige endoluminale Bestrahlung hat sich in der Palliativbehandlung zentraler Bronchialkarzinome als wirksam erwiesen und wird in Verbindung mit einer perkutanen Strahlenbehandlung als Methode der lokalen Dosiserhöhung ("Boost") zunehmend häufiger auch mit kurativer Zielsetzung angewendet. Bisherige Vorrichtungen zum endoluminalen Bestrahlen des Tracheal- und Bronchialbereiches besitzen den Nachteil, daß hohe örtliche Dosisbelastungen an der Tumoroberfläche oder der Bronchialschleimhaut auftreten können. Des weiteren darf die Ventilation der großen Stammbronchien bei den häufig ateminsuffizienten Patienten nicht durch eine zusätzliche künstliche Obstruktion beeinträchtigt werden. Es ist daher erforderlich, möglichst dünne Applikatorschläuche zu verwenden, die den Querschnitt der Stammbronchien nicht unnötig vermindern, jedoch liegt das Ende derartiger Applikatorschläuche mit der Strahlenquelle in der Regel einseitig an einer Wand des Hauptbronchus oder - bei Bestrahlung zentral sitzender Oberlappenkarzinome - an der Wand des Oberlappenbronchus an. Dies hängt mit der zum Einführen des Applikatorschlauchs und zum Einbringen der Strahlenquelle erforderlichen Mindeststeifheit zusammen.

Liegt das Ende des Applikatorschlauchs mit der Strahlenquelle an einer Wand eines Bronchus an, so treten bei einem Applikator mit 4 mm Durchmesser und einer mit einer Strahlenquelle beladenen Strecke von 40 mm bei einer Einzeldosis von 5 Gy, die auf eine Entfernung von 10 mm in bezug auf die Strahlenquelle berechnet ist, in der Mitte der bestrahlten Strecke Einzeldosen von 91 Gy an der Applikatoroberfläche auf. In einer Gewebetiefe von 5 mm beträgt die Dosis bei unmittelbarem Kontakt des Applikators mit der Schleimhaut oder Tumoroberfläche ungefähr 8 Gy. Die Bronchialwand wird hier von der 10 Gy-Isodose umschlossen und kleinvolumig mit 20 Gy und mehr belastet. Die gegenüberliegende Bronchialwand erhält bei einseitiger Anlage des Applikators nur Dosen zwischen 5 und 7 Gy, und in 5 mm Gewebetiefe liegt nur eine Einzeldosis von 2,5 Gy vor. Ein Tumor erhält an dieser Stelle daher kaum noch wirksame Dosen. Das einseitige Anliegen des Applikators an der Bronchialwand sowie der geringe Applikatordurchmesser führen daher zu einer äußerst ungleichmäßigen Dosisverteilung mit Überdosierung im Kontaktbereich und Unterdosierungen an der gegenüberliegenden Bronchialwand.

Schon kleinvolumige Überdosierungen im vorgeschädigten Bronchialsystem, z.B nach Operationen, Laserungen oder perkutanen Strahlenbehandlungen können zu schweren Komplikationen führen. Endoluminale Bestrahlungen im Bereich des tracheobronchialen Systems, wo zumeist zirkulär wachsende Tumore auftreten, setzen eine auf die Bronchialwand bezogene gleichmäßig rotationssymmetrische Dosisverteilung voraus.

Der Erfindung liegt daher die Aufgabe zugrunde, eine Vorrichtung zum Behandeln des Körperinneren zu schaffen, die bei kleinem Durchmesser des Applikators eine gleichmäßig rotationssymmetrische Dosisverteilung im Bestrahlungsbereich ergibt.

Gelöst wird diese Aufgabe bei einer Vorrichtung der eingangs erwähnten Art erfindungsgemäß durch mindestens einen ferngesteuerten, im Bereich der Strahlenquelle angeordneten, einen axialen Durchlaß freihaltenden Spreizkopf.

Die erfindungsgemäße Vorrichtung läßt sich im ungespreizten Zustand dank ihres geringen Durchmessers problemlos ins Körperinnere einführen und erlaubt es, am Ort der Bestrahlung eine gleichmäßige rotationssymmetrische Dosisverteilung zu erreichen. Wichtig ist, daß der zentrierende Spreizkopf einen ausreichend großen axialen Durchlaß freiläßt, wenn sich der Applikator im Lumen des Hauptbronchus oder der Trachea befindet, um eine Obstruktion der Atemwege zu vermeiden.

Vorzugsweise wird der axiale Durchlaß dadurch erreicht, daß der Spreiz- und Zentrierkopf radial spreizbare Stege aufweist, zwischen denen sich beim Spreizen ausreichend große Zwischenräume ergeben.

Zum Spreizen der Stege dient vorteilhafterweise eine axial geführte Zug- und/oder Druckvorrichtung, mit der die Stege des Spreizkopfes betätigt werden. Mit dieser Zug- und/oder Druckvorrichtung läßt sich der radiale Spreizweg der Weite des Bronchialsystems anpassen.

In besonders einfacher Weise läßt sich die erfindungsgemäße Vorrichtung mit radial spreizbaren Stegen verwirklichen, wenn mindestens drei Schlitze an einer oder mehreren Stellen gleichmäßig auf den Umfang eines elastisch verformbaren Schlauches angebracht werden. Zwischen den Schlitzen werden dadurch Schlauchmantelstreifen gebildet, die den radial spreizbaren Stegen entsprechen. Der elastisch verformbare Schlauch umgibt als äußerer Schlauch (Koaxialsystem) den Ausfahrschlauch für die Strahlenquelle.

Diese Mantelstreifen lassen sich mittels der Zug- und/oder Druckvorrichtung so stauchen, daß sich die Mantelstreifen zwischen den Schlitzen zu Stegen aufspreizen bzw. vorwölben. Je nach dem Ausmaß des Stauchens läßt sich der erforderliche Durchmesser des Spreizkopfes erreichen. Zu diesem Zweck sind die freien Enden des Ausfahrschlauchs und des elastisch verformbaren Schlauchs im Bereich der Strahlenquelle axial unverschiebbar miteinander verbunden und ist im Bereich der Belade- und/oder Lagerstation eine Vorrichtung zum axialen Verschieben des elastisch verformbaren Schlauchs relativ zum Ausfahrschlauch angeordnet.

Um ein ausreichendes Zentrieren über eine größere Länge zu erreichen, können mehrere axial versetzte Stege zwischen entsprechenden, axial versetzten Schlitzen liegen. Diese Stege wölben sich beim axialen Stauchen des Schlauches gleichzeitig im gewünschten Maße vor.

In ihrer einfachsten Form kann die Vorrichtung zum axialen Verschieben des elastisch verformbaren Schlauchs relativ zum Ausfahrschlauch aus einer Gewindestange am äußeren Ende des Ausfahrschlauchs und einer darauf geführten, sich am äußeren Ende des elastisch verformbaren Schlauchs abstützenden Mutter bestehen. Diese Mutter läßt sich von Hand verstellen, wobei eine auf der Gewindestange angeordnete Skaleneinteilung das Maß des radialen Vorwölbens der aufgestellten Stege angibt.

Es ist jedoch auch möglich, einen Drehantrieb für die Mutter und eine mit der Umdrehung der Mutter gekuppelte, das radiale Vorwölben der aufgestellten Stege wiedergebende Anzeigevorrichtung vorzusehen, so daß ein Betätigen von ferne möglich ist.

Mit der erfindungsgemäßen Vorrichtung läßt sich ein kontrolliertes Zentrieren der Strahlenquelle in situ ohne Beeinträchtigung der Ventilation erreichen. Um den Applikator einzubringen, wird zunächst ein Tubus endoskopisch am Abgang des Hauptbronchus plaziert. Durch den Tubus wird der Applikator an die gewünschte Stellung gebracht, wonach der Tubus zurückgezogen wird und der Applikator im Lumen des Hauptbronchus verbleibt. Das Aufstellen der Stege des Spreizkopfes läßt sich endoskopisch kontrollieren. Die zentrale Lage im Bronchusluftschatten läßt sich mittels einer eingebrachten Markierung im Röntgenbild nachweisen.

Mit der erfindungsgemäßen Vorrichtung ist eine endoluminale Bestrahlung eines zentralen Bronchialkarzinoms ohne die bisher beobachteten ernsthaften Komplikationen möglich. Es wurden nur noch selten akute fibrinoide Schleimhautnekrosen oder Tumoroberflächennekrosen beobachtet, die in den meisten Fällen nicht zu ernsthaften Komplikationen führten, jedoch eine, Unterbrechung der Behandlung erforderten. Schwere Komplikationen wie Bronchialwandperforationen oder Fisteln sind bei der Anwendung der erfindungsgemäßen Vorrichtung nicht mehr aufgetreten. Voraussetzung für die Anwendung eines Bronchialapplikators mit dem erfindungsgemäßen Zentrier- und Spreizkopf ist allerdings ein weitgehend offenes Bronchial- oder Tracheallumen, das durch Laserabtragen größerer stenosierender Tumoranteile geschaffen werden muß. Ein weiterer Vorteil der erfindungsgemäßen Vorrichtung liegt darin, daß sie sich an jede handelsübliche Belade- und/oder Lagerstation bzw. an jedes Nachladegerät anschließen läßt.

Die Erfindung wird nachstehend anhand eines in der Zeichnung dargestellten Ausführungsbeispiels des näheren erläutert. In der Zeichnung zeigen:

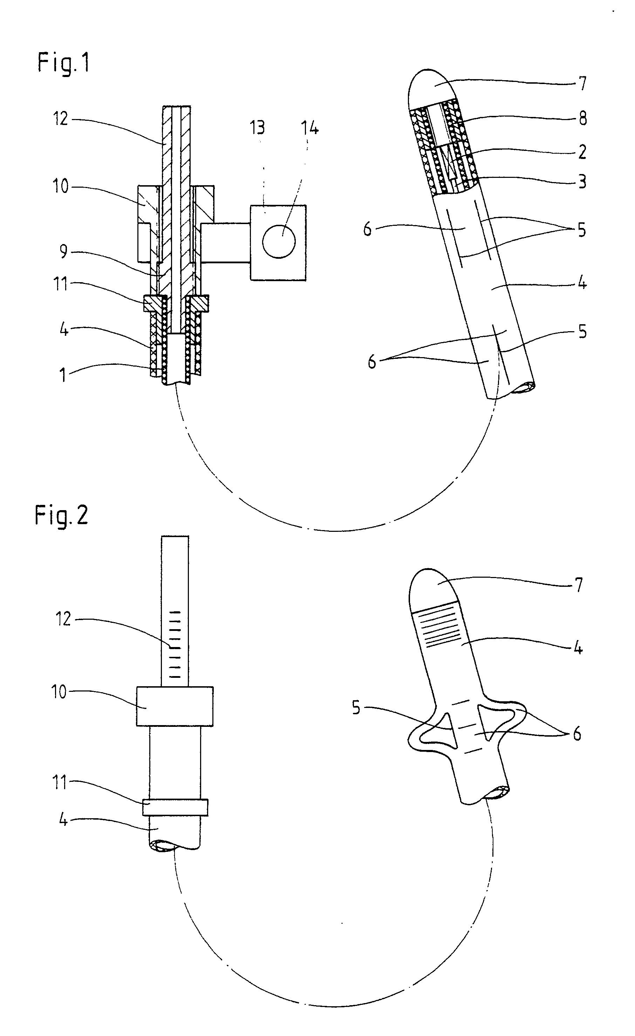

Fig. 1    eine erfindungsgemäße Vorrichtung mit Schnittdarstellung der Endbereiche im eingefahrenen Zustand der Zentriervorrichtung und

Fig. 2    eine Ansicht der erfindungsgemäßen Vorrichtung mit aufgestellten Stegen.

Ein Ausfahrschlauch 1, der in der Regel aus Polytetrafluoräthylen besteht, dient als Führung für eine mittels eines Kabels 3 im Ausfahrschlauch 1 verschiebbare Strahlenquelle 2. Der Ausfahrschlauch 1 und das Kabel 3 lassen sich mit einer nicht dargestellten Belade- und/oder Lagerstation kuppeln, die dazu dient, die Strahlenquelle 2 strahlengeschützt zu lagern und fernbedient durch den Ausfahrschlauch 1 in das Körperinnere des zu behandelnden Patienten einzubringen, ohne daß das behandelnde Personal durch die radioaktive Strahlung gefährdet wird.

Um den Ausfahrschlauch 1 herum ist ein elastisch verformbarer Schlauch 4 angeordnet, der ausreichend Spiel gegenüber dem Ausfahrschlauch 1 besitzt, so daß sich diese gegeneinander axial verschieben lassen. Der Ausfahrschlauch 1 und der elastisch verformbare Schlauch 4 sind über ein metallisches Endstück 7 axial unverschiebbar miteinander verbunden, z.B. durch in das Endstück eingedrehte Nuten 8, in denen sich das Ende des elastisch verformbaren Schlauchs 4 verkrallt. Im Bereich der Strahlenquelle 2 befinden sich im elastisch verformbaren Schlauch 4 achsparallel verlaufende Schlitze 5 gleichmäßig auf dem Umfang verteilt. Mindestens drei Schlitze 5, vorzugsweise aber vier und mehr können auf dem Umfang angeordnet sein, wodurch zwischen den Schlitzen 5 Schlauchstreifen 6 entstehen, die die radial gespreizten Stege des Spreizkopfes bilden.

Um diese Stege 6 aufzuspreizen, besitzt das freie Ende des Ausfahrschlauchs 1 ein Gewinderohr 9, auf das eine Mutter 10 geschraubt ist. Diese Mutter 10 stützt sich an einem Flansch 11 am Ende dem elastisch verformbaren Schlauchs 4 ab.

Wird nun die Mutter 10 gegen den Flansch 11 gedreht, wird das Gewinderohr 9 und der damit verbundene Ausfahrschlauch 1 aus dem elastisch verformbaren Schlauch 4 herausgezogen. Dadurch wird der elastisch verformbare Schlauch 4 gestaucht, was das Aufstellen der Stege 6 im Bereich der Schlitze 5 bewirkt.

Um das Ausmaß des radialen Aufspreizens der Stege 6 kontrollieren zu können, ist das Gewinderohr 9 mit einer Skaleneinteilung 12 versehen, die direkt auf den sich einstellenden Durchmesser der radial aufgestellten Stege 6 geeicht sein kann.

Im Falle einer Fernbedienung wird die Mutter 10 mit einem Drehantrieb 13 gekuppelt, die eine Anzeigevorrichtung 14 für das Ausmaß des radialen Spreizens der Stege 6 aufweisen kann.

Die Schlitze können je nach Außendurchmesser des elastisch verformbaren Schlauchs 4 eine Länge von 10 mm aufweisen und befinden sich im Abstand von 10 und 30 mm vom Endstück 7. Der Durchmesser des Spreizkopfes beträgt maximal 16 mm. Durch Veränderung der Schlitzlängen lassen sich unterschiedliche maximale Durchmesser des Spreizkopfes erreichen. Für die Hauptbronchien sind zweckmäßig zwei Spreizköpfe mit Schlitzlängen von 10 mm im Abstand von 15 bis 20 mm voneinander angeordnet. Für die Trachealbestrahlung ist ein Applikator mit mehr als zwei Spreizköpfen und größerem Durchmesser vorteilhaft.

**Patentansprüche**

1. Vorrichtung zum Behandeln des Körperinneren mit radioaktiver Strahlung mit einer in einem Ausfahrschlauch zum Behandlungsort verfahrbaren Strahlungsquelle, gekennzeichnet durch mindestens einen im Bereich der ausgefahrenen Strahlenquelle (2) angeordneten Spreizkopf (4, 5, 6).

2. Vorrichtung nach Anspruch 1, dadurch gekennzeichnet, daß der Spreizkopf (4, 5, 6) radial bewegliche Stege (6) aufweist.

3. Vorrichtung nach Anspruch 1 oder 2, dadurch gekennzeichnet, daß der Spreizkopf (4, 5, 6) mit einer axial geführten Zug- und/oder Druckvorrichtung (4, 9, 10, 11, 12) verbunden ist.

4. Vorrichtung nach Anspruch 3, dadurch gekennzeichnet, daß die radial spreizbaren Stege (6) zwischen achsparallel verlaufenden und gleichmäßig auf dem Umfang verteilten Schlitzen (5) in einem konzentrisch um den Ausfahrschlauch (1) angeordneten, elastisch verformbaren Schlauch (4) angeordnet sind.

5. Vorrichtung nach Anspruch 4, dadurch gekennzeichnet, daß die freien Enden des Ausfahrschlauchs (1) oder des elastisch verformbaren Schlauchs (4) im Bereich der ausgefahrenen Strahlenquelle (2) axial unverschiebbar miteinander verbunden sind und im Bereich der Belade- und/oder Lagerstation eine Vorrichtung (9, 10, 11, 12) zum axialen Verschieben des elastisch verformbaren Schlauchs (4) relativ zum Ausfahrschlauch (1) angeordnet ist.

6. Vorrichtung nach Anspruch 4 oder 5, dadurch gekennzeichnet, daß mehrere axial versetzte Stege (6) zwischen axial versetzten Schlitzen (5) im Ausfahrschlauch (1) liegen.

7. Vorrichtung nach Anspruch 4, 5, oder 6, gekennzeichnet durch eine Gewindestange (12) am äußeren Ende des Ausfahrschlauchs (4) und eine darauf angeordnete, sich am äußeren Ende (11) des elastisch verformbaren Schlauchs (4) abstützende Mutter (10).

8. Vorrichtung nach Anspruch 7, dadurch gekennzeichnet, daß auf der Gewindestange (12) eine der radialen Ausdehnung der aufgestellten Stege (6) entsprechende Skaleneinteilung (12) angeordnet ist,

9. Vorrichtung nach Anspruch 7, gekennzeichnet durch einen Drehantrieb (13) für die Mutter (10) und eine mit der Umdrehung der Mutter (10) gekuppelte, die radiale Ausdehnung der aufgestellten Stege (6) wiedergebende Anzeigevorrichtung (14).

Fig.1

Fig.2